# FASCICULE DE BREVET EUROPEEN

(11) **EP 2 468 220 B1**
(45) Date de publication et mention de la délivrance du brevet: **21.01.2015**
(21) Numéro de dépôt: 11194454.2
(22) Date de dépôt: 20.12.2011
(51) Int. Cl.: A61F 5/02

(54) **Dispositif de soutien lombaire**
Lendenstützvorrichtung
Lumbar support device

(30) Priorité: 21.12.2010 FR 1060987
(43) Date de publication de la demande: 27.06.2012
(73) Titulaire: Richard Freres, 42530 Saint Genest Lerpt (FR)
(72) Inventeur: Richard, Dominique, 42110 AUREC/LOIRE (FR); Faure, Didier, 43140 SAINT DIDIER EN VELAY (FR); Calet, Angélique, 42150 LA RICAMARIE (FR)
(74) Mandataire: Delorme, Nicolas

(56) Documents cités:
- EP-A1- 0 507 513
- EP-A1- 2 036 520
- FR-A1- 2 804 012
- US-A1- 2010 217 167

## Description

La présente invention se rapporte à un dispositif de soutien lombaire.

Il est connu de réaliser des ceintures de soutien lombaire comprenant une partie centrale destinée à être apposée sur le dos d'un utilisateur et dont les extrémités comportent des moyens de fermeture complémentaires du type à boucles et crochets. Ces ceintures permettent d'appliquer une pression sur la zone du dos de l'utilisateur sur laquelle la ceinture est apposée. Cette pression permet de soutenir et de soulager le dos de l'utilisateur. Les ceintures de soutien lombaire connues sont généralement réalisées en matière élastique et leur partie centrale comporte des moyens de rappel de posture, tels que des baleines disposées sensiblement dans la direction de l'axe antéropostérieur de l'utilisateur. Ces ceintures remplissent la plupart du temps, de manière satisfaisante, leur fonction de soutien lombaire tant que les activités de l'utilisateur restent modérées.

Cependant, lors d'activités plus intenses, par exemple un travail de force ponctuel, ces ceintures de soutien lombaire peuvent s'avérer insuffisantes pour soutenir et soulager le dos de l'utilisateur.

Il est également connu de réaliser des ceintures de soutien lombaire renforcées par une sangle de renfort fixée sur la ceinture de soutien. Par exemple, un dispositif de soutien lombaire selon le préambule de la revendication 1 est divulgué dans le document FR 2804012 A1.

La sangle de renfort exerce une pression supplémentaire au niveau de la zone du dos de l'utilisateur sur laquelle elle est apposée. Parfois, il peut être possible de régler la position de la sangle de renfort en hauteur. Ces ceintures munies de telles sangles s'avèrent satisfaisantes pour renforcer le soutien du dos de l'utilisateur, notamment lorsque celui-ci exécute un effort intense ponctuel.

Toutefois, les ceintures de soutien lombaire connues, munies de sangles de renfort, présentent l'inconvénient de n'offrir qu'un réglage en position complexe de la sangle de renfort lorsque ce réglage est possible.

Aussi la présente invention a-t-elle pour but de résoudre tout ou partie des inconvénients mentionnés ci-dessus.

Un but de l'invention est de proposer un dispositif de soutien lombaire permettant de soutenir le dos de l'utilisateur pour différents degrés d'activité.

Un autre but de l'invention est de proposer un dispositif de soutien lombaire permettant à l'utilisateur de sélectionner la zone du dos dont il souhaite renforcer le soutien.

L'invention a également pour but de proposer un dispositif de soutien lombaire avec une sangle dont le positionnement est à la fois simple et rapide.

A cet effet, la présente invention a pour objet un dispositif de soutien lombaire tel que défini dans la revendication 1, c'est-à-dire comportant une ceinture en matériau élastique présentant une partie centrale destinée à être positionnée sur la région lombaire d'un utilisateur et prolongée par deux parties latérales, les parties latérales étant munies de moyens de fermeture de la ceinture, et présentant un plan transversal P séparant la ceinture en une partie supérieure S et une partie inférieure I, une sangle dont les extrémités sont munies de moyens d'accrochage, et des moyens de liaison de la sangle et de la ceinture, caractérisé en ce que les moyens de liaison sont aptes à autoriser une rotation de 180° de la sangle par rapport à la ceinture autour d'un centre de rotation, la sangle étant asymétrique par rapport au plan transversal P passant par le centre de rotation, afin de permettre le positionnement de la sangle dans une position dite « haute » du dispositif de soutien lombaire, dans laquelle la pression de la ceinture et celle de la sangle s'ajoutent l'une à l'autre dans la région dorso-lombaire de l'utilisateur par la fermeture de la sangle, et une position dite « basse » du dispositif de soutien lombaire, dans laquelle la pression de la ceinture et celle de la sangle s'ajoutent l'une à l'autre dans la région lombo-sacrée de l'utilisateur par la fermeture de la sangle.

Ainsi, l'invention présente l'avantage de procurer un renfort de soutien du dos de l'utilisateur s'il le souhaite. Cela peut lui être particulièrement utile lors d'un effort intense ponctuel par exemple. L'invention permet également à l'utilisateur d'effectuer un réglage en position facile et rapide de la sangle. Dans la première position, l'utilisateur peut augmenter la pression exercée par la ceinture sur une zone dorso-lombaire. Dans la seconde position, l'utilisateur peut augmenter la pression exercée par la ceinture sur une zone lombo-sacrée. Il dispose ainsi de la faculté de renforcer le maintien de l'une ou l'autre de ces zones pour effectuer un effort les sollicitant particulièrement ou pour soulager des douleurs dorso-lombaires ou lombo-sacrées.

Selon une autre caractéristique du dispositif de soutien lombaire selon l'invention, la sangle comporte un élément central destiné à accueillir les moyens de liaison de la sangle et de la ceinture.

Préférentiellement, la sangle comporte deux bandes parallélépipédiques liées à l'une de leurs extrémités à l'élément central de manière à former un V.

De manière avantageuse, les moyens d'accrochage coopèrent avec les moyens de fermeture pour permettre la fermeture de la sangle.

Selon une autre caractéristique du dispositif de soutien lombaire selon l'invention, les moyens de fermeture comportent chacun une zone supérieure et une zone inférieure situées respectivement dans la partie supérieure (S) et la partie inférieure (I), et les moyens d'accrochage coopèrent avec les zones supérieures lorsque la sangle est fermée dans une position dite « haute » et avec les zones inférieures lorsque la sangle est fermée dans une position dite « basse ».

De manière avantageuse, les moyens de liaison sont du type non permanent, de sorte que la sangle soit amovible.

Cette caractéristique présente l'avantage de permettre le retrait de la sangle lorsque celle-ci n'est pas utilisée. Ainsi, la sangle n'encombre pas l'utilisateur.

De manière avantageuse, les moyens de liaison comprennent un bouton pression.

Cette caractéristique présente l'avantage de permettre la rotation de la sangle par rapport à la ceinture et de pouvoir solidariser ou désolidariser aisément la sangle et la ceinture en fonction des besoins de l'utilisateur.

Préférentiellement, la partie centrale de la ceinture comporte une pluralité de points de fixation constituant autant de centres de rotation possibles de la sangle par rapport à la ceinture.

Ainsi, l'utilisateur peut effectuer un premier réglage en hauteur de la position de la sangle en choisissant le point de fixation sur lequel la fixer.

Selon une autre caractéristique du dispositif de soutien lombaire selon l'invention, les moyens de liaison comprennent au moins un point de couture entre la sangle et la ceinture.

Selon une autre caractéristique du dispositif de soutien lombaire selon l'invention, les moyens de liaison comprennent un rivet.

Selon une autre caractéristique du dispositif de soutien lombaire selon l'invention, la partie centrale de la ceinture comporte des moyens de rappel de posture.

On comprendra mieux les buts, aspects et avantages de la présente invention d'après la description donnée ci-après d'un mode de réalisation de la présente invention, donnée à titre d'exemple non limitatif, en se référant aux dessins annexés dans lesquels :
- La figure 1 est une vue de face d'un dispositif de soutien lombaire selon un mode particulier de réalisation de l'invention, dans une première position d'utilisation ;
- La figure 2 est une vue de face d'un dispositif de soutien lombaire selon un mode particulier de réalisation de l'invention, dans une seconde position d'utilisation ;
- La figure 3 est une vue de face d'un dispositif de soutien lombaire selon un mode particulier de réalisation de l'invention, illustrant une phase transitoire permettant de passer de la position illustrée par la figure 1 à la position illustrée par la figure 2.

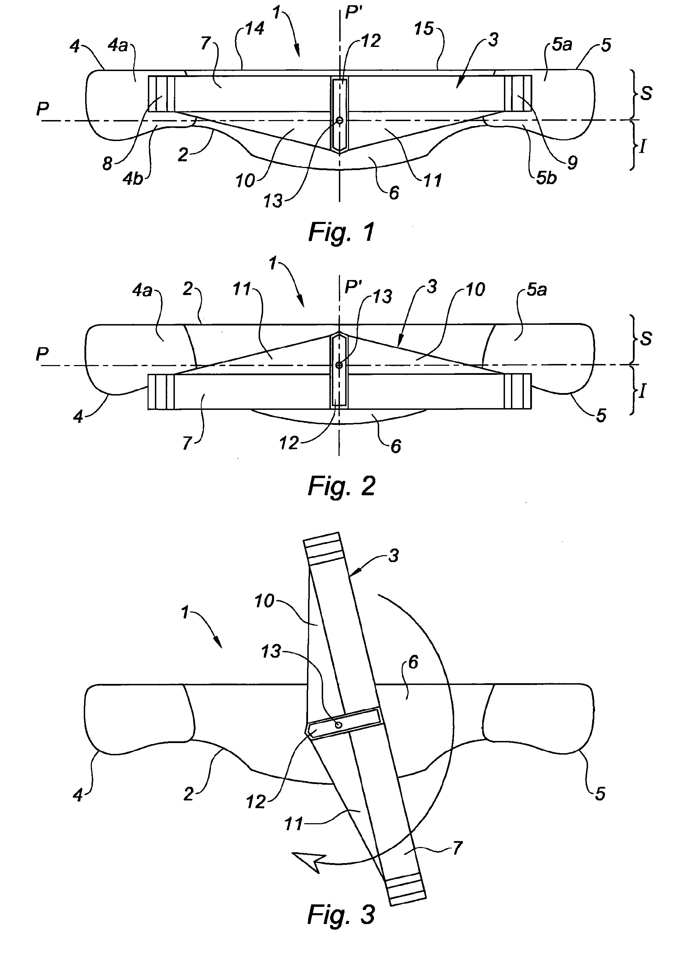

La figure 1 représente un dispositif de soutien lombaire 1 comportant une ceinture 2 et une sangle 3. La ceinture 2 est en tissu élastique. La ceinture 2 présente un plan transversal P qui la divise en une partie supérieure S et une partie inférieure I, le plan transversal P passant par un centre de rotation 13 comme cela est visible sur la figure 1. La ceinture présente aussi un plan médian P' passant par le centre de rotation 13 et la divisant en deux parties latérales 14,15. En fonctionnement, quand la ceinture 2 est positionnée sur un utilisateur, le plan transversal P est sensiblement parallèle au plan anatomique transversal de l'utilisateur et le plan médian P' est sensiblement parallèle au plan anatomique médian de l'utilisateur. La ceinture 2 comporte également une partie centrale 6. La partie centrale 6 de la ceinture 2 est prolongée par les parties latérales 14,15 destinées à enserrer l'abdomen d'un utilisateur ; les parties latérales comportent chacune des moyens de fermeture 4,5 de la ceinture 2. Dans le mode de réalisation décrit, les moyens de fermeture 4,5 sont complémentaires, du type textile à boucles et crochets. La partie supérieure S et la partie inférieure I de la ceinture 2 définissent au niveau des moyens de fermeture 4,5 des zones supérieures 4a, 5a et des zones inférieures 4b, 5b.

La partie centrale 6 est munie de moyens de rappel de posture, non représentés sur les figures. Les moyens de rappel de posture peuvent être réalisés au moyen de baleines disposées selon un axe sensiblement perpendiculaire au plan transversal P.

La sangle 3 comporte une bande rectangulaire 7 dont chaque extrémité est munie de moyens d'accrochage 8,9. Dans le mode de réalisation décrit, les moyens d'accrochage 8,9 sont du type textile à boucles ou crochets.

La sangle 3 comporte en outre deux bandes parallélépipédiques 10,11 et un élément central 12. Chaque bande parallélépipédique 10,11 possède une extrémité liée à l'une des extrémités de la bande rectangulaire 7 et une autre extrémité liée à l'élément central 12 ou à l'autre bande parallélépipédique 10 ou 11, de sorte que les bandes parallélépipédiques 10,11 forment un V, comme cela est représenté sur les différentes figures.

Lorsque la sangle 3 est en position d'utilisation, l'élément central 12 est disposé dans une direction sensiblement perpendiculaire au plan transversal P. L'élément central 12 est destiné à accueillir des moyens de liaison de la sangle 3 sur la ceinture 2. Les moyens de liaison sont aptes à permettre une rotation de 180° entre la sangle 3 et la ceinture 2. Les moyens de liaison forment ainsi une liaison pivot entre la sangle 3 et la ceinture 2. Dans le mode de réalisation décrit, les moyens de liaison sont réalisés par un point de couture entre la ceinture 2 et l'élément central 12 de la sangle 3. Dans une variante non représentée, les moyens de liaison peuvent être réalisés directement entre la ceinture 2 et la bande rectangulaire 7 ou entre la ceinture 2 et l'une des bandes parallélépipédiques 11,12. Les moyens de liaison peuvent également être réalisés par un système de bouton pression ou par un rivet. Les moyens de liaison comportent le centre de rotation 13 par lequel passe le plan transversal P et autour duquel s'effectue la rotation de la sangle 3 par rapport à la ceinture 2, comme cela est représenté sur la figure 3.

La sangle 3 est asymétrique par rapport au plan transversal P passant par le centre de rotation 13. Cette caractéristique, en combinaison avec la faculté de rotation de la sangle 3 par rapport à la ceinture 2, permet de définir deux positions d'utilisation de la sangle 3.

Dans une première position d'utilisation, dite « haute », représentée sur la figure 1, la sangle 3 couvre une zone essentiellement située au-dessus du plan transversal P. Dans cette position d'utilisation, la fermeture de la sangle 3 permet de renforcer la pression exercée par la ceinture 2 sur la région dorso-lombaire de l'utilisateur.

Dans une seconde position d'utilisation, dite « basse », visible sur la figure 2, la sangle 3 couvre une zone essentiellement située en-deçà du plan transversal P. Dans cette position d'utilisation, la fermeture de la sangle 3 permet de renforcer la pression exercée par la ceinture 2 sur la région lombo-sacrée de l'utilisateur.

L'utilisateur peut donc régler la sangle 3 de manière à la positionner dans deux positions différentes, et ce d'une manière très simple puisqu'une rotation de 180° dans le sens horaire ou anti-horaire suffit à positionner correctement la sangle 3. Le dispositif de soutien lombaire 1 selon le mode de réalisation décrit s'affranchit ainsi de tout problème de positionnement complexe.

Dans le mode de réalisation décrit, les moyens d'accrochage 8,9 permettent la fermeture de la sangle 3 sur les moyens de fermeture 4,5 de la ceinture 2. Plus précisément, dans la position d'utilisation dite « haute », les moyens d'accrochage 8,9 se fixent sur les zones supérieures 4a, 5a des moyens de fermeture 4,5. Dans la position d'utilisation dite « basse », les moyens d'accrochage 8,9 se fixent sur les zones inférieures 4b, 5b des moyens de fermeture 4,5. La fermeture de la sangle 3 obtenue par la fixation des moyens d'accrochage 8,9 sur les moyens de fermeture 4,5 permet la fermeture de la sangle 3 et donc le renforcement du soutien déjà apporté par la ceinture 2.

Dans une variante de réalisation non représentée, les moyens d'accrochage 8,9 peuvent être complémentaires, du type textile à boucles et crochets, et se fixer l'un avec l'autre pour permettre la fermeture de la sangle 3 sur elle-même, indépendamment des moyens de fermeture 4,5 de la ceinture 2.

De plus, les moyens de liaison de la sangle 3 et de la ceinture 2 peuvent être du type non permanent. Ainsi, la sangle 3 peut être amovible. Cela permet de retirer la sangle 3 lorsque l'activité de l'utilisateur reste modérée et ne nécessite pas de renfort de soutien supplémentaire à celui exercé par la ceinture 2.

Dans une variante non représentée, la partie centrale 6 de la ceinture 2 comporte une pluralité de points de rotation disposés à différentes hauteurs sur la partie centrale 6 et sensiblement compris dans le plan médian P'. L'utilisateur peut choisir de lier la sangle 3 à l'un de ces points de rotation. Le point de rotation sélectionné se confond alors avec le centre de rotation 13. L'avantage de disposer de plusieurs points de rotation, et par conséquent de plusieurs centres de rotation 13 possibles, est de permettre à l'utilisateur d'effectuer un premier réglage simple de la position de la sangle 3 par rapport à la ceinture 2. Les points de rotation de la partie centrale 6 de la ceinture 2 peuvent être réalisés par des parties mâles ou femelles de boutons pression coopérant respectivement avec les parties femelles ou mâles de ces boutons pression situées sur l'élément central 12.

Bien entendu, l'invention n'est nullement limitée au mode de réalisation décrit ci-dessus, ce mode de réalisation n'ayant été donné qu'à titre d'exemple. Des modifications restent possibles, notamment du point de vue de la constitution des divers éléments ou par la substitution d'équivalents techniques, sans pour autant sortir du domaine de protection de l'invention.

Ainsi, la sangle 3 peut être élastique ou non. Lorsque la sangle 3 est élastique, celle-ci peut comporter plusieurs tissus présentant des modules d'élasticité différents, de sorte que la sangle 3 exerce une pression non uniforme sur le dos de l'utilisateur. En particulier, l'élément central 12 peut présenter un module d'élasticité différent de celui des bandes parallélépipédiques 10 et 11.

Les moyens de liaison entre la sangle 3 et la ceinture 2 peuvent être réalisés par des boutons cousus sur la partie centrale de la ceinture 2 ou l'élément central 12 et destinés à être insérés au travers d'une fente ménagée respectivement dans l'élément central 12 ou la partie centrale 6 de la ceinture 2.

## Revendications

1. Dispositif de soutien lombaire (1) comportant une ceinture (2) en matériau élastique présentant une partie centrale (6) destinée à être positionnée sur la région lombaire d'un utilisateur et prolongée par deux parties latérales (6a,6b), les parties latérales (6a,6b) étant munies de moyens de fermeture (4,5) de la ceinture (2), et présentant un plan transversal (P) séparant ladite ceinture (2) en une partie supérieure (S) et une partie inférieure (I), une sangle (3) dont les extrémités sont munies de moyens d'accrochage (8,9), et des moyens de liaison de la sangle (3) et de la ceinture (2), **caractérisé en ce que** les moyens de liaison sont aptes à autoriser une rotation de 180° de la sangle (3) par rapport à la ceinture (2) autour d'un centre de rotation (13), la sangle (3) étant asymétrique par rapport au plan transversal (P) passant par le centre de rotation (13), afin de permettre le positionnement de la sangle (3) dans une position dite « haute » du dispositif de soutien lombaire (1), dans laquelle la pression de la ceinture (2) et celle de la sangle (3) s'ajoutent l'une à l'autre dans la région dorso-lombaire de l'utilisateur par la fermeture de la sangle (3), et une position dite « basse » du dispositif de soutien lombaire (1), dans laquelle la pression de la ceinture (2) et celle de la sangle (3) s'ajoutent l'une à l'autre dans la région lombo-sacrée de l'utilisateur par la fermeture de la sangle (3).

2. Dispositif de soutien lombaire (1) selon la revendication 1, **caractérisé en ce que** la sangle (3) comporte un élément central (12) destiné à accueillir les moyens de liaison de la sangle (3) et de la ceinture (2).

3. Dispositif de soutien lombaire (1) selon la revendication 2, **caractérisé en ce que** la sangle (3) comporte deux bandes parallélépipédiques (10,11) liées à l'une de leurs extrémités à l'élément central (12) de manière à former un V.

4. Dispositif de soutien lombaire (1) selon l'une quelconque des revendications 1 à 3, **caractérisé en ce que** les moyens d'accrochage (8,9) coopèrent avec les moyens de fermeture (4,5) pour permettre la fermeture de la sangle (3).

5. Dispositif de soutien lombaire (1) selon la revendication 4, **caractérisé en ce que** les moyens de fermeture (4,5) comportent chacun une zone supérieure (4a,5a) et une zone inférieure (4b,5b) situées respectivement dans la partie supérieure (S) et la partie inférieure (I), et **en ce que** les moyens d'accrochage (8,9) coopèrent avec les zones supérieures (4a,5a) lorsque la sangle (3) est fermée dans une position dite « haute » et avec les zones inférieures (4b,5b) lorsque la sangle (3) est fermée dans une position dite « basse ».

6. Dispositif de soutien lombaire (1) selon l'une quelconque des revendications 1 à 5, **caractérisé en ce que** les moyens de liaison sont du type non permanent, de sorte que la sangle (3) soit amovible.

7. Dispositif de soutien lombaire (1) selon l'une quelconque des revendications 1 à 6, **caractérisé en ce que** les moyens de liaison comprennent un bouton pression.

8. Dispositif de soutien lombaire (1) selon l'une quelconque des revendications 1 à 7, **caractérisé en ce que** la partie centrale de la ceinture (2) comporte une pluralité de points de fixation constituant autant de centres de rotation (13) possibles de la sangle (3) par rapport à la ceinture (2).

9. Dispositif de soutien lombaire (1) selon l'une quelconque des revendications 1 à 5, **caractérisé en ce que** les moyens de liaison comprennent un point de couture entre la sangle (3) et la ceinture (2).

10. Dispositif de soutien lombaire (1) selon l'une quelconque des revendications 1 à 5, **caractérisé en ce que** les moyens de liaison comprennent un rivet.

11. Dispositif de soutien lombaire (1) selon l'une quelconque des revendications 1 à 10, **caractérisé en ce que** la partie centrale (6) de la ceinture (2) comporte des moyens de rappel de posture.

## Patentansprüche

1. Lendenstützvorrichtung (1), umfassend einen Gürtel (2) aus elastischem Material, der einen Mittelteil (6) hat, der dazu vorgesehen ist, auf dem Lendenbereich eines Benutzers positioniert zu werden, und durch zwei Seitenteile (6a, 6b) verlängert ist, wobei die Seitenteile (6a, 6b) mit Mitteln (4, 5) zum Schließen des Gürtels (2) ausgestattet sind, und eine Querebene (P) hat, die den besagten Gürtel (2) in einen oberen Teil (S) und einen unteren Teil (I) unterteilt, einen Gurt (3), dessen Enden mit Kopplungsmitteln (8, 9) ausgestattet sind, und Mittel zum Verbinden des Gurts (3) und des Gürtels (2), **dadurch gekennzeichnet, dass** die Verbindungsmittel dazu fähig sind, eine 180°-Drehung des Gurts (3) in Bezug auf den Gürtel (2) um einen Drehpunkt (13) herum zuzulassen, wobei der Gurt (3) asymmetrisch in Bezug auf die Querebene (P) ist, die durch den Drehpunkt (13) hindurch verläuft, um die Positionierung des Gurts (3) in einer so genannten "hohen" Position der Lendenstützvorrichtung (1), in der der Druck des Gürtels (2) und der des Gurts (3) im Lenden-Rückenbereich des Benutzers durch das Schließen des Gurts (3) miteinander kombiniert werden, und in einer so genannten "tiefen" Position der Lendenstützvorrichtung (1), in der der Druck des Gürtels (2) und der des Gurts (3) im Kreuzbein-Lendenbereich des Benutzers durch das Schließen des Gurts (3) miteinander kombiniert werden, zu ermöglichen.

2. Lendenstützvorrichtung (1) nach Anspruch 1, **dadurch gekennzeichnet, dass** der Gurt (3) ein mittleres Element (12) umfasst, das dazu vorgesehen ist, die Mittel zum Verbinden des Gurts (3) und des Gürtels (2) aufzunehmen.

3. Lendenstützvorrichtung (1) nach Anspruch 2, **dadurch gekennzeichnet, dass** der Gurt (3) zwei parallelepipedische Streifen (10, 11) umfasst, die an einem ihrer Ende mit dem mittleren Element (12) so verbunden sind, dass die ein V bilden.

4. Lendenstützvorrichtung (1) nach einem der Ansprüche 1 bis 3, **dadurch gekennzeichnet, dass** die Kopplungsmittel (8, 9) mit den Schließmitteln (4, 5) zusammenwirken, um das Schließen des Gurts (3) zu ermöglichen.

5. Lendenstützvorrichtung (1) nach Anspruch 4, **dadurch gekennzeichnet, dass** die Schließmittel (4, 5) jeweils eine obere Zone (4a, 5a) und eine untere Zone (4b, 5b) umfassen, die sich in dem oberen Teil (S) bzw. dem unteren Teil (I) befinden, und dass die Kopplungsmittel (8, 9) mit den oberen Zonen (4a, 5a) zusammenwirken, wenn der Gurt (3) in einer so genannten "hohen" Position geschlossen wird, und mit den unteren Zonen (4b, 5b) zusammenwirken, wenn der Gurt (3) in einer so genanten "tiefen" Position geschlossen wird.

6. Lendenstützvorrichtung (1) nach einem der Ansprüche 1 bis 5, **dadurch gekennzeichnet, dass** die Verbindungsmittel von der nicht dauerhaften Art sind, so dass der Gurt (3) lösbar ist.

7. Lendenstützvorrichtung (1) nach einem der Ansprüche 1 bis 6, **dadurch gekennzeichnet, dass** die Verbindungsmittel einen Druckknopf aufweisen.

8. Lendenstützvorrichtung (1) nach einem der Ansprüche 1 bis 7, **dadurch gekennzeichnet, dass** der Mittelteil des Gürtels (2) mehrere Befestigungspunkte umfasst, die so viele Drehpunkte (13) wie möglich des Gurts (3) in Bezug auf den Gürtel (2) bilden.

9. Lendenstützvorrichtung (1) nach einem der Ansprüche 1 bis 5, **dadurch gekennzeichnet, dass** die Verbindungsmittel eine Nahtstelle zwischen dem Gurt (3) und dem Gürtel (2) umfassen.

10. Lendenstützvorrichtung (1) nach einem der Ansprüche 1 bis 5, **dadurch gekennzeichnet, dass** die Verbindungsmittel eine Niete umfassen.

11. Lendenstützvorrichtung (1) nach einem der Ansprüche 1 bis 10, **dadurch gekennzeichnet, dass** der Mittelteil (6) des Gürtels (2) Haltungswiederherstellungsmittel umfasst.

## Claims

1. A lumbar support device (1) including a belt (2) made of elastic material presenting a central portion (6) intended to be positioned on the lumbar region of a user and extended with two side portions (6a, 6b), the side portions (6a, 6b) being provided with means (4, 5) for closing the belt (2), and presenting a transverse plane (P) separating said belt (2) into an upper portion (S) and a lower portion (I), a strap (3) the ends of which are provided with attachment means (8, 9), and means for connecting the strap (3) and the belt (2), **characterized in that** the connecting means are adapted to allow a rotation of 180° of the strap (3) relative to the belt (2) around a center of rotation (13), the strap (3) being asymmetrical relative to the transverse plane (P) passing by the center of rotation (13), in order to allow the positioning of the strap (3) in a position called "high" position of the lumbar support device (1), in which the pressure of the belt (2) and that of the strap (3) are added to each other in the dorso-lumbar region of the user by the closing of the strap (3), and a position called "low" position of the lumbar support device (1), in which the pressure of the belt (2) and that of the strap (3) are added to each other in the lumbo-sacral region of the user by the closing of the strap (3).

2. The lumbar support device (1) according to claim 1, **characterized in that** the strap (3) includes a central element (12) intended to receive the means for connecting the strap (3) and the belt (2).

3. The lumbar support device (1) according to claim 2, **characterized in that** the strap (3) includes two parallelepiped strips (10, 11) connected at one of their ends to the central element (12) so as to form a V-shape.

4. The lumbar support device (1) according to any one of claims 1 to 3, **characterized in that** the attachment means (8, 9) cooperate with the closing means (4, 5) in order to allow the closing of the strap (3).

5. The lumbar support device (1) according to claim 4, **characterized in that** the closing means (4, 5) include each an upper area (4a, 5a) and a lower area (4b, 5b) located respectively in the upper portion (S) and the lower portion (I), and **in that** the attachment means (8, 9) cooperate with the upper areas (4a, 5a) when the strap (3) is closed in a position called "high" position and with the lower areas (4b, 5b) when the strap (3) is closed in a position called "low" position.

6. The lumbar support device (1) according to any one of claims 1 to 5, **characterized in that** the connecting means are of a non-permanent type, so that the strap (3) is removable.

7. The lumbar support device (1) according to any one of claims 1 to 6, **characterized in that** the connecting means comprise a snap fastener.

8. The lumbar support device (1) according to any one of claims 1 to 7, **characterized in that** the central portion of the belt (2) includes a plurality of fixing points constituting as many centers of rotation (13) as possible of the strap (3) relative to the belt (2).

9. The lumbar support device (1) according to any one of claims 1 to 5, **characterized in that** the connecting means comprise a stitch between the strap (3) and the belt (2).

10. The lumbar support device (1) according to any one of claims 1 to 5, **characterized in that** the connecting means comprise a rivet.

11. The lumbar support device (1) according to any one of claims 1 to 10, **characterized in that** the central portion (6) of the belt (2) includes posture correcting means.
